# EUROPEAN PATENT APPLICATION

(11) **EP 4 711 172 A1**
(43) Date of publication of application: **18.03.2026**
(21) Application number: 24200806.8
(22) Date of filing: 17.09.2024
(51) Int. Cl.: B60K 35/10, A61B 5/18, B60K 35/22, B60K 35/26, B60K 35/28, G06V 20/59, B60K 28/02

(54) **VEHICLE OCCUPANT CAPTURING SYSTEM COMPRISING ADDITIONAL OUTPUT OPTION FOR FACE YOGA INFORMATION, AS WELL AS METHOD**

(71) Applicant: Valeo Comfort and Driving Assistance, 94000 Créteil (FR)
(72) Inventor: Qi, Jing, 74321 Bietigheim-Bissingen (DE); Bozbayir, Evin, 74321 Bietigheim-Bissingen (DE); Reilhac, Patrice, 74321 Bietigheim-Bissingen (DE)
(74) Representative: Jauregui Urbahn, Kristian

(57) **Abstract**

One aspect of the invention relates to a vehicle occupant capturing system (6) comprising
- at least one capturing unit (7) for capturing a face (9) of a vehicle occupant (4);
- an evaluation unit (10) for evaluating the captured information (12) of the face (9);
- a communication module (11) for communicating with an Occupant Monitoring face analysis module (13), wherein by the Occupant Monitoring face analysis module (13), digital information as to at least one muscle-training-exercise is provided to the vehicle occupant (4);
- an output unit (14), by which digital information as to at least one muscle-training-exercise depending on the evaluated information of the face (9) is output to the vehicle occupant (4) for implementing exercises of face muscular movement and/or muscular training of face muscles and/or face relaxation exercises and/or face stress relief.

## Description

One aspect of the invention relates to a vehicle occupant capturing system. A further aspect of the invention relates to a method for capturing and assessing a vehicle occupant of a vehicle.

Electronic vehicle occupant capturing systems are known. They commonly comprise at least one capturing unit to be able to capture a vehicle occupant. For example, such a capturing unit may be a camera. Thus, for example a driver of the vehicle may be captured. For example, thereby the attention state may be recognized. Thus, the vehicle occupant capturing system may also be or support a tiredness detection system. Moreover, such a vehicle occupant capturing system may for example also be used for triggering an airbag or a corresponding assistance system. Depending for example on the position of a head of a vehicle occupant the triggering of the airbag may be controlled.

In the named known vehicle occupant capturing systems, however, the functionality in each case is limited. They are commonly conceived only for one of the named functions each.

Particularly in modern vehicles, in which assistance systems are increasingly capable of taking over also the guiding of the vehicle at least semi-autonomously, a vehicle occupant therefore may increasingly dedicate themselves to other activities during their stay in the vehicle.

It is the objective of the present invention to provide a vehicle occupant capturing system, a vehicle, and a method, in which a varied use of such a system is facilitated.

This task is solved by a vehicle occupant capturing system, a vehicle, as well as a method according to the independent claims.

One aspect of the invention relates to a vehicle occupant capturing system. The vehicle occupant capturing system comprises at least one capturing unit for capturing a face of a vehicle occupant. Moreover, the vehicle occupant capturing system also comprises an evaluation unit. The same according to the intended purpose is configured to evaluate the captured information of the face. The vehicle occupant capturing system moreover comprises a communication module for communicating with an Occupant Monitoring face analysis module, hereinafter face yoga module. By the face yoga module in particular digital information as to at least one muscle-training-exercise, hereinafter face yoga, is provided. The vehicle occupant capturing system moreover comprises an output unit, by which digital information as to face yoga depending on the evaluated information of the face is capable of being output or is output. Thus, a vehicle occupant capturing system is provided, which in addition to its primary function now also comprises a secondary function. The primary function may therein be the capturing of at least one part of the body of the vehicle occupant, in particular a face, in order to thereby provide information for an assistance system of the vehicle. This assistance system may for example be a tiredness detection system or an airbag system. The secondary function in this connection is here in particular not linked to the functionality of a further electronic assistance system of a vehicle. Rather, it may here preferably be used for assisting the vehicle occupant themselves with regard to their current and/or future well-being. Thereby a vehicle occupant capturing system is provided, which may be used multi-functionally with regard to the capturing of a face.

By the specific communication module it is thus facilitated to present digital information for relaxation and/or face stress relief and/or health care particularly for the face of a vehicle occupant. Thus, by the vehicle occupant capturing system it is now also facilitated, particularly during a stay of a vehicle occupant in the vehicle, by possibly specific face training, namely face yoga, to improve their current state and/or also lastingly a future state. Here, in a very interactive way a vehicle occupant capturing system may be extended to improve the time of a vehicle occupant in the vehicle for their current and/or future state of health, in particular that of their face, and thus possibly also a state of attention of the vehicle occupant for the guiding of the vehicle.

In an embodiment the vehicle occupant capturing system comprises at least one muscle-training-exercise mode, hereinafter face yoga mode, and comprises at least one assistance system mode that is different therefrom. In the assistance system mode depending on the captured information of the face a fitness to drive and/or a degree of attention of the vehicle occupant is capable of being determined. In particular, thus, in the assistance system mode for example also a tiredness mode may be comprised.

In an embodiment the vehicle occupant capturing system is configured in such a way that digital information as to face yoga depending on the evaluated information of the face is capable of being output or is output only in the face yoga mode. This also means that the vehicle occupant capturing system also with this implementation of multiple functions complies with high safety requirements. This means for example that the face yoga mode is only activated if the vehicle is guided autonomously by the assistance system. In particular when the driver of the vehicle need not take over the driving and thus at best needs to be available for immediate takeover of the driving of the vehicle, then the face yoga mode may be activated.

However, it is also possible that the face yoga mode comprises various mode levels. Then, in an embodiment, it may be possible that the face yoga mode is also capable of being activated if an assistance system mode of the vehicle occupant capturing system is active. This is because then also in such constellations, in which the vehicle occupant, then in particular a driver of the vehicle, has to perform the driving of the vehicle partly manually and with raised attention, the face yoga mode may then be activated at the same time in a low mode level. For example, then possibly also acoustic or only acoustically determined digital information as to face yoga may be output, which the vehicle occupant concerned, here the driver of the vehicle, may perceive and as a consequence of which perform corresponding face yoga exercises themselves. However, it is also possible that in a then correspondingly low mode level also optically digital information as to face yoga is output on the output unit. This is in particular the case if the output unit also comprises a display unit, such as for example a display or the like. Precisely when this optical information is very low-threshold, but for instance very little and easy to understand, it may also be displayed simultaneously with the assistance system mode of the vehicle occupant capturing system on the output unit.

In an embodiment the face yoga module forms integral part of the vehicle occupant capturing system. The face yoga module may be an Occupant Monitoring face analysis software module and/or a hardware module.

In an embodiment a captured information of the face is one of the following:
- complexion;
- skin structure;
- wrinkle position of wrinkles in the face;
- wrinkle shape, that is depth and/or length and/or course of a wrinkle;
- a facial expression;
- movements of at least subportions of the face; and
- facial symmetry.

This means that these may be decision criteria as to whether and, if applicable, which digital information as to face yoga is required or advantageous. In particular, the communication module is also configured to transfer this information of the face yoga module to the output unit. In particular, it is transferred by this communication module to the output unit.

In an embodiment the output unit is configured to output digital information as to face yoga depending on the evaluated information of the face in images, that is in static images, or as video.

In an embodiment at least subportions of the face of the captured vehicle occupant are shown and information of the face yoga module is displayed as superimposition to the subportions on the output unit. Thereby, optically a very simple and intuitively perceivable and plausible presentation of the information is facilitated. The respective vehicle occupant may then process corresponding information very fast and, if applicable, also certain procedures or specifications as to face yoga may then also be easily followed by the vehicle occupant. Thus, also the training of the face on the basis of this digital information as to face yoga is facilitated in a particularly simple and easy to understand way. Both the current mobility of the face and thus also the relaxation and/or face stress relief and/or attention is thus improved, as well as perspectively a firmer facial appearance is achievable

In an embodiment the subportions and/or information of the face yoga module are output dynamically and/or in real time for a movement of the face.

Thus the plausible and particularly continuous and exact performance of specific movements of the face, in particular of face muscles, is facilitated. Thereby the effect, which is to be achieved by the digital information as to face yoga at the output unit, is particularly efficient.

In an embodiment information of the face yoga module consists in exercises for face muscular movement and/or muscular training of face muscles and/or face relaxation exercises and/or face stress relief.

In an embodiment, but not limited to, there can be an evolution over time of the face and/or muscles of the face, which can provide further auxiliary evaluated information to the vehicle occupant based on the evolution of the face and/or the current state of the face, for example having auxiliary evaluated information like tiredness and/or aging, which can be in line with the theme of personalization for the vehicle occupant and/or vehicle.

In an embodiment, but not limited to, there can overlay information provided on the vehicle interior digital cluster and/or infotainment system. The overlay information can be colored line visualizations that instruct the vehicle occupant about at least one muscle-training-exercise to be conducted via the hands and/or fingers of the vehicle occupant. The at least one muscle-training-exercise can be content instructions for skin massage on the occupant's face by their hands and/or fingers. The overlay instructions on the vehicle interior display device (eg: digital mirror, digital cluster, infotainment system) can provide visual aids to the vehicle occupant on techniques to contort the face for muscular movement and/or muscular training of face muscles and/or face relaxation exercises and/or face stress relief by way of the hands and/or fingers of the vehicle occupant.

A further aspect of the invention relates to a vehicle comprising a vehicle occupant capturing system according to the above-named aspect or an advantageous embodiment thereof. A further aspect of the invention relates to a method for capturing and assessing a vehicle occupant of a vehicle. The method preferably comprises the following steps:
- capturing a face of a vehicle occupant by at least one capturing unit of a vehicle occupant capturing system;
- evaluating the captured information of the face by an evaluation unit;
- outputting digital information as to at least one muscle-training-exercise provided via a communication module depending on the evaluated information of the face by an output unit.

Advantageous embodiments of the vehicle occupant capturing system are to be regarded as advantageous embodiments of the method. In particular, aspects of the vehicle occupant capturing system are facilitated by the corresponding units so that the method steps resulting therefrom are given.

In particular, the vehicle also comprises an electronic assistance system, by which the guiding of the vehicle is at least semi-autonomously assisted. This assistance system by interacting with the vehicle occupant capturing system may form an overall system. It may also be integral part of the vehicle occupant capturing system.

Embodiments of the invention are explained in more detail with reference to schematic drawings in the following. The figures show:
- Fig. 1: an embodiment of a schematic vehicle comprising a schematic vehicle occupant capturing system; and
- Fig. 2: an embodiment of a schematic vehicle occupant capturing system.

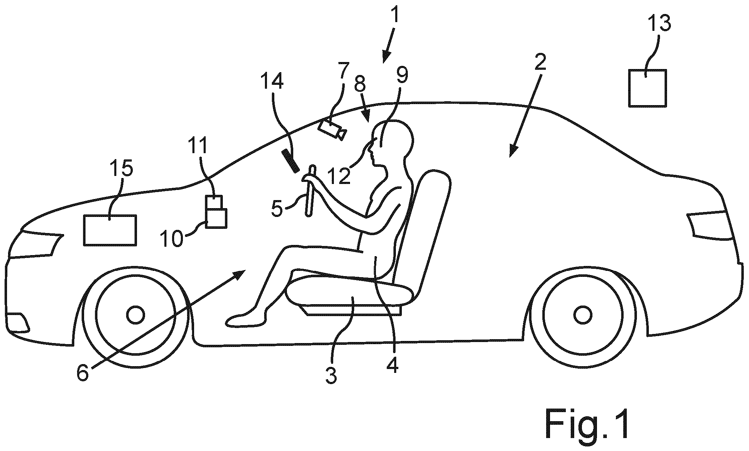

The Fig. 1 in a schematic view shows an embodiment of a vehicle according to the invention comprising an embodiment of a vehicle occupant capturing system according to the invention.

In Fig. 1 a vehicle 1 is shown in simplified representation. The vehicle 1 may be a motor vehicle. It may in particular be a passenger car or a truck or a bus.

The vehicle 1 comprises a passenger compartment 2. The same according to the intended purpose is envisaged for vehicle occupants to be seated in when driving the vehicle 1. The vehicle 1 moreover in the shown embodiment comprises a driver seat 3. On the same a vehicle occupant 4 is shown and positioned schematically. The vehicle occupant 4 here is a vehicle driver.

The vehicle 1 comprises moreover a steering element 5. The same may for example be a steering wheel. The vehicle occupant 4 in their function as a vehicle driver actuates this steering element 5 when manually steering the vehicle 1.

Moreover, in Fig. 1 an embodiment of an electronic vehicle occupant capturing system 6 is shown. The vehicle occupant capturing system 6 comprises at least one capturing unit 7. The same may for instance be a camera. The camera may be sensitive in particular in the visible spectral range and/or in the infrared range. The capturing unit 7 is oriented in such a way that it may capture the vehicle occupant 4 at least partially by its detection region. In particular, here a specific part of the body 8 is envisaged for capturing. Here the part of the body 8 is the face 9. Moreover, the vehicle occupant capturing system 6 is an evaluation unit 10. This may be a software and/or hardware component. The evaluation unit 10 is configured to evaluate the information 12 of the face 9 captured by the at least one capturing unit 7. In particular, the vehicle occupant capturing system 6 also comprises a communication module 11. The same may also be a software and/or hardware component. The communication module 11 is configured to communicate with an Occupant Monitoring face analysis module, hereinafter face yoga module 13. By the face yoga module 13 digital information as to at least one muscle-training-exercise, hereinafter face yoga, is provided. The face yoga module 13 may be arranged external to the vehicle 1 or, however, also in the vehicle 1. It may be a software module or a hardware module. A communication interface between the communication module 11 and the face yoga module 13 may be configured to be wireless or wired.

Preferably, the vehicle occupant capturing system 6 may also comprise at least one output unit 14. The output unit 14 may be configured for acoustic and/or optical output of information. The output unit 14 is configured to output digital information as to face yoga depending on the evaluated information of the face 9. In particular this information is displayed and/or acoustically output on this output unit 14.

The vehicle 1 may, moreover, preferably comprise at least one electronic assistance system 15, which assists the guiding of the vehicle 1 at least semi-autonomously. The assistance system 15 may communicate with the vehicle occupant capturing system 6. It is also possible that an overall system comprises the at least one assistance system 15 and the vehicle occupant capturing system 6.

In an embodiment the vehicle occupant capturing system 6 comprises at least one muscle-training-exercise mode, hereinafter face yoga mode, and at least one assistance mode that is different therefrom as operating states. In this assistance system mode depending on the captured information of the face 9 a fitness to drive or a degree of attention of the vehicle occupant 4 is capable of being determined.

The vehicle occupant capturing system 6 is configured in such a way that digital information as to face yoga depending on the evaluated information of the face 9 in the face yoga mode, in an embodiment is capable of being output, in particular is output, only in this face yoga mode to the output unit 14. On the basis of the information of the face 9 captured by the at least one capture unit 7 in particular the complexion and/or skin structure and/or a wrinkle position and/or a wrinkle shape and/or a facial expression and/or a facial symmetry and/or a movement of at least subportions of the face 9 are evaluated.

In an embodiment it is envisaged that the output unit 14 is configured to output digital information as to face yoga depending on the evaluated information of the face 9 in images and/or as video. It is possible that subportions of the face 9 are visually displayed on an output unit 14 configured as display unit. In particular then also information of the face yoga module is preferably displayed as superimposition to these subportions of the face 9 on this display unit. It is possible that the subportions of the face 9 and/or information of the face yoga module for movement of the face 9 are output dynamically and/or in real time at the output unit 14.

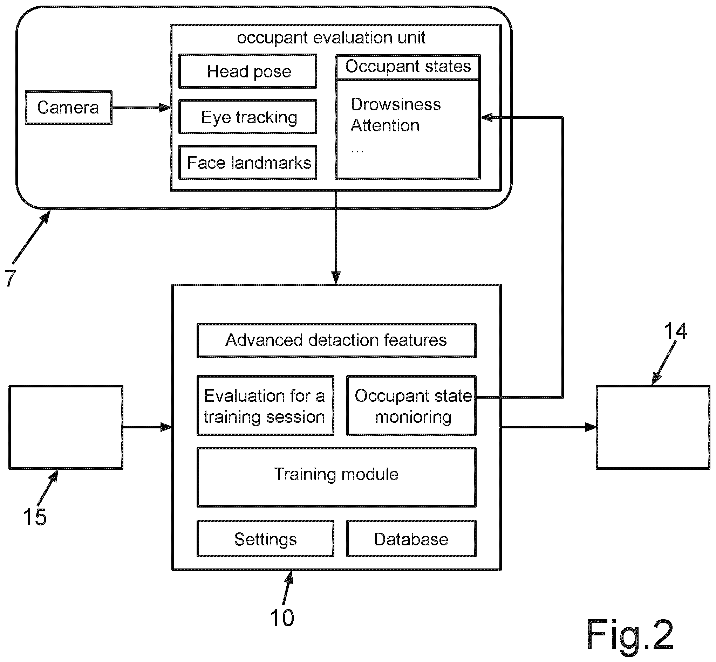

Fig. 2 shows an embodiment of a schematic vehicle occupant capturing system 6. The capturing unit 7 is also able to determine, for example, the head pose and/or the eye tracking and/or face landmarks and/or several driver states. Thanks to these detection, especially in real-time, the evaluation unit 10 is able to proceed, especially on the basis of a training session, to evaluate and provide driver state information, to record screenshots or video of specific face events and to manage a training Yoga face session.

The head pose of the occupant 4 is related to the yaw angle and/or the pitch angle and/or the roll angle.

Embodiments for the face landmarks are nose root left, nose root right, right and/or left eyebrow inner, right and/or left eyebrow outer, right and/or left eye top, right and/or left pupil, right and/or left eye inner, right and/or left eye outer, right and/or left eye bottom, right and/or left nose alar top, right and/or left nose alar out tip, right and/or left side of the mouth, under lip top, under lip bottom, upper lip top, upper lip bottom.

In addition to this information, in an embodiment the evaluation unit 10 has more advanced detection features for complexion, skin structure, wrinkle position, wrinkle shape, facial expression, movements of at least subportions of the face and facial symmetry.

## Claims

1. Vehicle occupant capturing system (6) comprising
- at least one capturing unit (7) for capturing a face (9) of a vehicle occupant (4);
- an evaluation unit (10) for evaluating the captured information (12) of the face (9);
- a communication module (11) for communicating with an Occupant Monitoring face analysis module (13), wherein by the Occupant Monitoring face analysis module (13), digital information as to at least one muscle-training-exercise is provided to the vehicle occupant (4);
- an output unit (14), by which digital information as to at least one muscle-training-exercise depending on the evaluated information of the face (9) is output to the vehicle occupant (4) for implementing exercises of face muscular movement and/or muscular training of face muscles and/or face relaxation exercises and/or face stress relief.

2. Vehicle occupant capturing system (6) according to claim 1, wherein the vehicle occupant capturing system (6) comprises at least one muscle-training-exercise mode and at least one assistance system mode that is different therefrom and in which depending on the captured information (12) of the face (9) a fitness to drive and/or a degree of attention of the vehicle occupant (4) is capable of being determined.

3. Vehicle occupant capturing system (6) according to claim 2, wherein the vehicle occupant capturing system (6) is configured in such a way that digital information as to at least one muscle-training-exercise depending on the evaluated information of the face (9) is output only in the at least one muscle-training-exercise mode.

4. Vehicle occupant capturing system (6) according to any one of the preceding claims, wherein the Occupant Monitoring face analysis module (13) is integral part of the vehicle occupant capturing system (6).

5. Vehicle occupant capturing system (6) according to any preceding claim, wherein a captured information (12) of the face (9) is at least one of the following:
- complexion;
- skin structure;
- wrinkle position, wrinkle shape;
- facial expression;
- movements of at least subportions of the face;
- facial symmetry.

6. Vehicle occupant capturing system (6) according to any preceding claim, wherein the output unit (14) is configured to output digital information as to at least one muscle-training-exercise depending on the evaluated information of the face (9) in images or as video.

7. Vehicle occupant capturing system (6) according to claim 6, wherein at least subportions of the face (9) of the captured vehicle occupant (4) are displayed and information of the Occupant Monitoring face analysis module (13) is displayed as superimposition to the subportions on the output unit (14).

8. Vehicle occupant capturing system (6) according to claim 7, wherein the subportions and/or the information of the Occupant Monitoring face analysis module (13) are output dynamically and/or in real time for movement of the face (9).

9. Vehicle occupant capturing system (6) according to any one of the preceding claims, wherein information of the Occupant Monitoring face analysis module (13) is exercises for face muscular movement and/or muscular training of face muscles and/or face relaxation exercises and/or face stress relief.

10. Vehicle occupant capturing system (6) according to any preceding claim, wherein auxiliary evaluated information is provided to the vehicle occupant (4) based on the evolution over time of the face and/or muscles of the face and/or the current state of the face.

11. Vehicle occupant capturing system (6) according to claim 10, wherein the auxiliary evaluated information comprises at least one of tiredness and/or aging.

12. Vehicle occupant capturing system (6) according to any preceding claim, wherein the digital information comprises overlay information colored line visualizations that instruct the vehicle occupant (4) about at least one muscle-training-exercise to be conducted via the hands and/or fingers of the vehicle occupant (4), which is provided on an interior digital cluster and/or infotainment system and/or digital mirror of a vehicle (1).

13. Vehicle occupant capturing system (6) according to any preceding claim, wherein the at least one muscle-training-exercise comprises content instructions and/or visual aids to the vehicle occupant (4) on techniques to contort the face for muscular movement and/or muscular training of face muscles and/or face relaxation exercises and/or face stress relief and/or skin massage by way of the hands and/or fingers of the vehicle occupant (4).

14. Vehicle (1) comprising a vehicle occupant capturing system (6) according to any preceding claim.

15. Method for capturing and assessing a vehicle occupant (4) of a vehicle (1), comprising the following steps:
- capturing a face of a vehicle occupant (4) by at least one capturing unit (7) of a vehicle occupant capturing system (6);
- evaluating the captured information (12) of the face (9) by an evaluation unit (10);
- outputting digital information as to at least one muscle-training-exercise provided via a communication module (11) depending on the evaluated information of the face (9) by an output unit (14).
